Europäisches Patentamt

European Patent Office

Office européen des brevets

(19)

(11) Numéro de publication: **0 361 987**

**A1**

## (12) DEMANDE DE BREVET EUROPEEN

(21) Numéro de dépôt: 89400587.5

(22) Date de dépôt: 02.03.89

(51) Int. Cl.5: **G06K 9/00 , A61B 5/103**

(30) Priorité: 04.03.88 FR 8802803

(43) Date de publication de la demande:
04.04.90 Bulletin 90/14

(84) Etats contractants désignés:
**DE GB IT**

(71) Demandeur: **ETAT FRANCAIS représenté par le Ministre Délégué des Postes et Télécommunications (CENTRE NATIONAL D'ETUDES DES TELECOMMUNICATIONS) 38-40 rue du Général Leclerc**
**F-92131 Issy-les-Moulineaux(FR)**

(72) Inventeur: **Andre, Catherine**
**34, rue du Clos Herbert**
**F-14000 Caen(FR)**
Inventeur: **Duval, Sylvain**
**La Besnardière La Sauvagère**
**F-61600 La Ferte Mace(FR)**

(74) Mandataire: **Plaçais, Jean-Yves et al**
**Cabinet Netter, 40, rue Vignon**
**F-75009 Paris(FR)**

(54) **Dispositif de formation d'une image d'une surface en relief, notamment une empreinte digitale.**

(57) Une empreinte digitale (ED) est en contact sur la surface d'appui d'un prisme à réflexion totale (3). Celui-ci est éclairé par un faisceau incident (FI) issu d'une source lumineuse (S) dont le spectre est étroit. Le faisceau réfléchi (FR) traverse un dispositif optique (4) comportant des moyens propres à corriger l'anamorphose du motif correspondant à ladite surface en relief obtenu par réflexion sur la surface d'appui, cette anamorphose étant occasionnée par l'inclinaison de la surface d'appui relativement au faisceau incident (FI) et propres à redresser le plan de l'image (ED2) dans une direction tendant à le rendre parallèle à une section droite du faisceau réfléchi (FR) qui l'atteint. Un premier dispositif optique (2) comporte des moyens de convergence propres à faire converger le faisceau source (FS) en un point constituant une source sensiblement ponctuelle.

FIG.1

# Dispositif de formation d'une image d'une surface en relief, notamment une empreinte digitale.

L'invention concerne la formation d'une image d'une surface en relief en contact avec une surface d'appui d'un milieu transparent homogène. Cette image comporte des zones de coloration différentes représentant les creux et les saillies de la surface en relief.

Au sens de la présente invention, le terme "saillie" représente les parties de la surface en relief en contact avec la surface d'appui, tandis que le terme "creux" représente les parties de cette surface en relief non en contact avec la surface d'appui.

L'invention convient particulièrement aux empreintes digitales bien qu'elle puisse s'appliquer à toute surface en relief pour laquelle on souhaite différencier les creux des saillies.

Une application particulièrement intéressante de l'invention est l'authentification d'empreintes digitales. Les systèmes actuels d'authentification d'empreintes digitales comprennent un dispositif optique de formation d'une image de l'empreinte à authentifier, associé à un dispositif de traitement informatique de l'image ainsi formée. Ce dispositif informatique saisit l'image formée, la traite et la code pour la comparer à une empreinte digitale de référence codée de façon similaire. S'il y a coïncidence entre l'image formée et la référence il y a authentification.

Un support particulièrement bien adapté à l'enregistrement de ces références est une carte à mémoire. Un utilisateur potentiel introduit sa carte à mémoire dans le système d'authenfication et pose son doigt sur un capteur optique du dispositif optique. Le système compare alors les données présentes dans la carte à celles qu'il extrait de l'image formée par le dispositif optique. Comme l'espace mémoire dans une carte est réduit, le système doit être capable d'y stocker des informations exhaustives sur l'empreinte non ambiguës. En conséquence les logiciels utilisés n'auront de réelle efficacité que grâce à une excellente qualité de l'image formée.

Les dispositifs optiques actuels comprennent généralement une source lumineuse générant un faisceau lumineux, suivie d'une lentille convergente destinée à produire un faisceau incident. Ce faisceau incident pénètre à l'intérieur d'un milieu homogène transparent pour venir se réfléchir sur une surface d'appui sur laquelle la surface en relief est en contact. Cette surface d'appui est inclinée par rapport à la direction d'incidence de façon à réfléchir totalement les parties du faisceau incident qui frappent la surface d'appui au niveau des creux, les parties du faisceau incident qui frappent la surface d'appui au niveau des saillies étant en partie absorbées dans la peau. Le faisceau réfléchi sort du milieu transparent et traverse une lentille convergente destinée à la mise au point de l'image de la surface en relief.

Plusieurs inconvénients résultent d'un tel dispositif. Le faisceau réfléchi l'est sous un angle identique à l'angle d'incidence si bien que le plan de formation de l'image obtenue n'est pas parallèle à la section droite de ce faisceau, mais incliné de l'angle d'incidence. Si l'on utilise une caméra, par exemple munie d'une matrice à transfert de charge (CCD"charge coupled device"), il faut orienter cette matrice convenablement pour récupérer le plan image. Cependant, lorsque l'objectif de la caméra est long il y a perte de lumière du faisceau réfléchi à l'intérieur de l'objectif ce qui se traduit par un éclairage non uniforme et des difficultés de mise au point.

En outre, puisque le plan image est incliné par rapport à la section droite du faisceau réfléchi qui l'atteint, la caméra restitue une image qui semble comprimée latéralement. Ainsi la fréquence des crêtes, par exemple, a augmenté suivant une seule direction. Si l'empreinte à comparer a tourné par rapport à l'empreinte de référence, la compression fait que, même après correction de la rotation, les deux images venant pourtant du même doigt paraîtront toujours différentes d'où des erreurs possibles à l'authentification.

Pour pallier ces inconvénients on peut utiliser des milieux transparents à embossage, ou en forme de tuile. La surface d'appui n'est pas plane mais elle épouse la courbure du doigt. Cependant, le plan image se trouvant sur une surface courbe, on ne peut pas faire de mise au point statique; il faut donc utiliser nécessairement une méthode de balayage mécanique ce qui rend le système délicat à synchroniser et trop lent.

De plus, les milieux transparents possédant une surface d'appui sensiblement plane nécessitent un éclairage puissant quand la source utilisée n'est pas cohérente. Généralement on utilise des lampes classiques à filament qui ne sont pas des sources ponctuelles mais des sources étendues et peu directives. On obtient alors un éclairement non uniforme avec de fortes variations de contrastes dans l'image obtenue.

La présente invention vient apporter une amélioration de l'image obtenue dans des dispositifs utilisant des milieux transparents possédant une surface d'appui sensiblement plane.

Un but de l'invention est de corriger l'effet de compression latérale occasionné par l'inclinaison de la surface d'appui et de redresser le plan image dans une direction tendant à le rendre parallèle à la section droite du faisceau réfléchi qui l'atteint.

Un autre but de l'invention est d'améliorer la netteté et le contraste de l'image obtenue.

L'invention a encore pour but d'utiliser une source lumineuse économique n'exigeant pas de précautions particulières d'emploi et n'occasionnant pas de fortes variations de contrastes dans l'image.

Un autre but de l'invention est de limiter les aberrations de sphéricité occasionnées par le dispositif de façon à améliorer encore la netteté et le contraste de l'image obtenue.

L'invention a donc pour objet un dispositif de formation d'une image d'une surface en relief, notamment une empreinte digitale, comprenant :
- une source lumineuse générant un faisceau source lumineux,
- une premier montage optique produisant un faisceau incident à partir dudit faisceau source,
- un milieu homogène transparent possédant un dioptre d'entrée par lequel le faisceau incident pénètre à l'intérieur dudit milieu selon une direction principale d'incidence, une surface d'appui sensiblement plane sur laquelle la surface en relief est en contact, cette surface d'appui étant inclinée relativement à la direction principale d'incidence de façon à réfléchir totalement une partie du faisceau incident, le faisceau réfléchi sortant du milieu selon une direction principale de réflexion par l'intermédiaire d'un dioptre de sortie et comprenant un motif correspondant à ladite surface en relief, avec une anamorphose occasionnée par l'inclinaison de la surface d'appui,
- un deuxième montage optique recevant le faisceau réfléchi et propre à former ladite image, caractérisé en ce que la source lumineuse a un spectre étroit
- en ce que le premier dispositif optique comporte des moyens de convergence propres à faire converger le faisceau source en un point constituant une source auxiliaire sensiblement ponctuelle et
- en ce que le deuxième dispositif optique comporte des moyens de correction propres à redresser le plan de ladite image dans une direction tendant à le rendre parallèle à une section droite du faisceau réfléchi qui l'atteint, ces moyens de correction étant en outre propres à compenser l'anamorphose dudit motif.

Selon un mode de réalisation de l'invention, le plan contenant les directions principales d'incidence et de réflexion, dit plan optique, est sensiblement perpendiculaire à la surface d'appui et les moyens de correction comprennent avantageusement une lentille cylindrique convergente dont l'axe géométrique longitudinal est sensiblement orthogonal audit plan optique.

De préférence, la distance focale de la lentille cylindrique est supérieure à la distance maximale séparant son centre optique de la surface d'appui et les moyens de correction comprennent en outre de préférence, une lentille sphérique convergente placée en aval de la lentille cylindrique.

Dans un but d'amélioration de la netteté de l'image, les moyens de correction peuvent comprendre un filtre spatial disposé au niveau du segment de convergence de la lentille cylindrique.

Selon un mode de réalisation de l'invention, les moyens de convergence comprennent une première lentille sphérique convergente propre à faire converger le faisceau source au point constituant la source auxiliaire, cette première lentille sphérique convergente étant suivie d'une deuxième lentille sphérique convergente située à une distance de la source auxiliaire au moins égale à sa distance focale.

Très avantageusement, la source lumineuse est une diode électroluminescente.

Selon un mode préféré de réalisation, le milieu homogène transparent est un prisme de section droite triangulaire rectangle, dont les deux côtés perpendiculaires sécants constituent respectivement les dioptre d'entrée et de sortie et dont le côté joignant les deux côtés perpendiculaires sécants constitue la surface d'appui, les dioptres d'entrée et de sortie étant respectivement sensiblement parallèles aux sections droites des faisceaux incident et réfléchi qui les traversent.

Dans un autre mode de réalisation de l'invention, les premier et deuxième montages optiques comprennent respectivement des premiers et deuxièmes moyens de renvoi des faisceaux incident et réfléchi les traversant. Ainsi il est possible de loger le dispositif selon l'invention dans un boîtier compact.

D'autres avantages et caractéristiques de l'invention apparaîtront à la lecture de la description détaillée ci-après et des dessins annexés sur lesquels :
- la figure 1 est un synoptique schématique du dispositif selon l'invention,
- la figure 2 est une vue de dessus schématique d'un premier mode de réalisation du dispositif selon l'invention,
- la figure 3 est une représentation schématique illustrant la réflexion du faisceau incident, et,
- la figure 4 est une représentation schématique d'un second mode de réalisation du dispositif selon l'invention.

Les dessins comportant pour l'essentiel des éléments de caractère certain font partie intégrante de la description. A ce titre ils pourront servir non seulement à mieux faire comprendre la description détaillée ci-après mais aussi contribuer le cas échéant à la définition de l'invention.

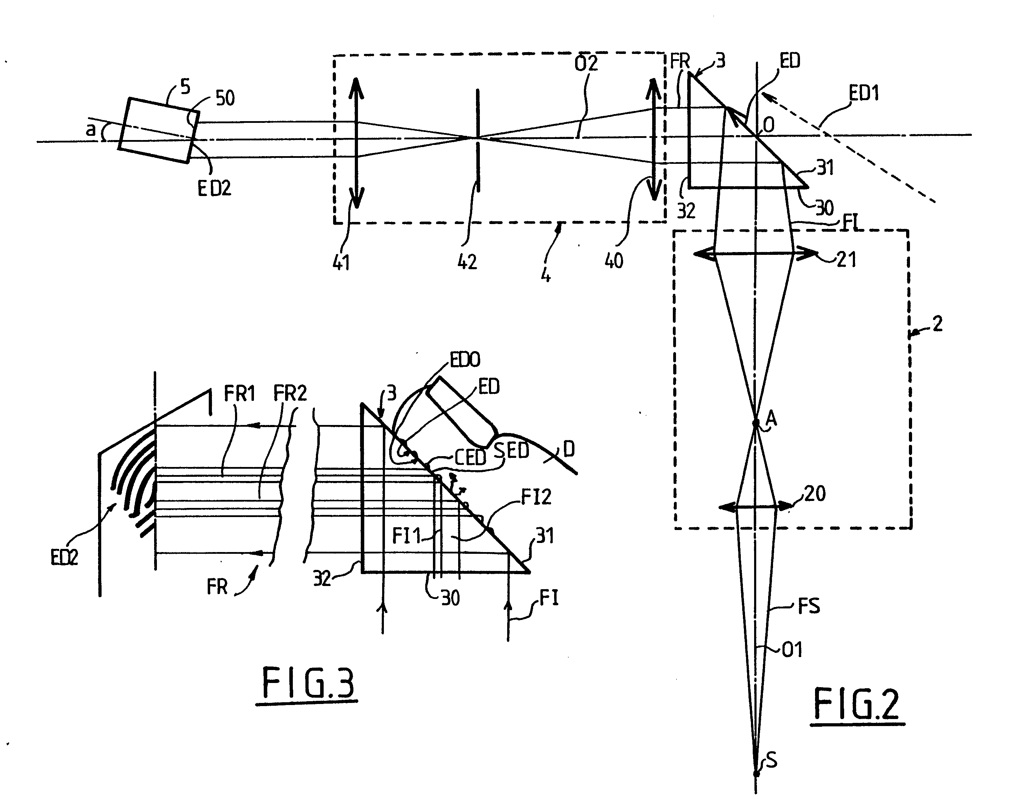

En se référant à la figure 1, on voit qu'un dispositif 1 selon l'invention, comprend de façon générale une source lumineuse S générant un faisceau source lumineux FS. Un premier montage optique 2 produit un faisceau incident FI à partir du faisceau source FS. Ce faisceau incident FI pénètre à l'intérieur d'un milieu homogène transparent 3 possédant une surface d'appui sur laquelle est en contact l'empreinte digitale ED d'un doigt D. Ainsi qu'il sera expliqué ci-après, le faisceau incident FI se réfléchit sur cette surface d'appui selon un faisceau réfléchi FR qui pénètre dans un deuxième dispositif optique 4 lequel forme une image ED2 de l'empreinte digitale ED. Cette image ED2 est reçue sur un récepteur 5.

On va maintenant décrire en se référant plus particulièrement à la figure 2, un premier mode de réalisation du dispositif selon l'invention. La disposition des différents éléments constitutifs de ce dispositif, représentée sur cette figure 2, est celle utilisée par le demandeur pour la mise au point de l'invention. Les valeurs numériques fournies ci-après ne le sont bien entendu qu'à titre indicatif. Elles sont de plus fournies avec une incertitude relative moyenne de 5% environ. Il va de soi que ce premier mode de réalisation du dispositif permet d'illustrer le principe général de l'invention.

La source lumineuse S est une diode électroluminescente du type de celles fabriquées par la société japonaise STANLEY. Cette diode électroluminescente a un angle d'ouverture de 9 degrés et émet une onde dont la longueur d'onde est comprise entre environ 0,62 et environ 0,71 micron. Cette source lumineuse a donc un spectre étroit.

L'utilisation d'une diode électroluminescente est particulièrement avantageuse dans cette application par rapport à des diodes laser par exemple. En effet ces dernières sont sensibles aux brusques variations de tension de leur alimentation ce qui peut les détruire et il faut donc concevoir une alimentation stabilisée pour l'utilisation de telles diodes laser. De plus, leur puissance de sortie dépend de la température et leur durée de vie décroît exponentiellement avec l'augmentation de la température. Il est donc nécessaire de prévoir en outre un système de stabilisation en température. Ces précautions d'emploi ont conduit le Demandeur à préférer l'utilisation d'une diode électroluminescente facile d'emploi, très résistante et consommant peu.

Le premier dispositif optique 2 comprend une première lentille sphérique convergente 20 d'un diamètre de 2,4 cm, dont le centre optique est situé à 9 cm de la source lumineuse S et dont la distance focale est de 2 cm. Le premier dispositif optique 2 comprend également une deuxième lentille sphérique convergente 21, d'un diamètre de 4 cm, située à 8,5 cm de la première lentille convergente 20. Sa distance focale est de 5 cm. Le faisceau source FS arrivant sur la première lentille convergente 20 converge en un point A constituant une source auxiliaire sensiblement ponctuelle. Cette source auxiliaire A est située à 2,6 cm du centre optique de la première lentille sphérique 20. Ainsi il convient de remarquer que la distance séparant la source auxiliaire A du centre optique de la deuxième lentille sphérique 21 est supérieure à la distance focale de cette dernière. Les raisons de cet agencement seront expliquées ci-après. Le dispositif optique 2 produit donc à la sortie de la deuxième lentille sphérique 21 le faisceau incident FI. La source lumineuse S et les centres optiques respectifs des première et deuxième lentilles convergentes sont alignés selon un premier axe optique 01.

Le milieu homogène transparent 3 est un prisme de section droite triangulaire rectangle dont les deux côté perpendiculaires sécants constituent respectivement un dioptre d'entrée 30 et un dioptre de sortie 32. Le côté joignant les deux côtés perpendiculaires 30 et 32 constitue la surface d'appui 31 sur laquelle est en contact l'empreinte digitale ED. L'axe optique 01 est sensiblement perpendiculaire au dioptre plan 30 et la surface d'appui 31 est inclinée d'un angle de 45 degrés par rapport à cet axe optique 01. Le point de concours 0 de l'axe optique 01 et de la surface d'appui 31 est situé à 4 cm du centre optique de la deuxième lentille sphérique convergente 21. Ce prisme est en verre avec un indice optique égal à environ 1,5. La surface d'appui 31 du prisme est suffisamment grande pour pouvoir supporter le doigt D.

En aval de ce prisme se trouve le deuxième dispositif optique 4. Celui-ci possède un deuxième axe optique 02 coplanaire avec le premier axe optique 01, ce plan étant dit plan optique, et concourant également au point 0. Ce deuxième dispositif optique comprend une lentille cylindrique convergente 40, située à 3,5 cm du point 0, dont l'axe géométrique longitudinal est sensiblement orthogonal au plan optique. Cette lentille cylindrique n'agira donc que sur un objet situé dans un plan parallèle au plan optique, et n'aura aucun effet sur un objet situé dans un plan perpendiculaire à ce plan optique. La lentille cylindrique 40 a une distance focale de 8 cm, supérieure à la distance maximale séparant son axe optique de la surface en relief, et fournit une image virtuelle ED1.

Le deuxième dispositif optique 4 comprend également en aval de cette lentille cylindrique 40, une lentille sphérique convergente 41 dont le centre optique est situé à 10 cm de la lentille cylindrique 40. Cette lentille sphérique a une distance focale de 4 cm, et un diamètre de 3,15 cm. Le centre optique de cette lentille sphérique est bien entendu situé sur l'axe optique 02 qui coupe également l'axe optique de la lentille cylindrique 40.

Entre la lentille cylindrique et la lentille sphérique, est disposé au niveau du segment de convergence de la lentille cylindrique 40, un filtre spatial 42 dont les fonctions seront expliquées ci-après. Ce filtre spatial est constitué d'un masque en forme de fente de largeur réglable pouvant aller jusqu'à 3 mm et d'une hauteur de 1 cm. La hauteur étant bien entendu une dimension prise dans le plan perpendiculaire au plan optique. Le plan de cette fente est situé à 4,3 cm du centre optique de la lentille sphérique convergente 41.

L'image ED2 de l'empreinte digitale ED formée par ce deuxième dispositif optique est dans un plan dont le point d'intersection avec l'axe optique 02 se trouve à environ 5,5 cm du centre optique de la lentille sphérique convergente 41. La trace du plan de l'image ED2 sur le plan optique est inclinée d'un angle a.

Dans le plan de formation de l'image ED2 est placée une matrice CCD 50 faisant partie d'un récepteur CCD classique. Les dimensions de cette matrice CCD sont de 8,8 mm x 6,6 mm environ.

Le fonctionnement de ce dispositif va maintenant être décrit en se référant également à la figure 3. La diode électroluminescente S envoie donc le faisceau source FS sur la première lentille sphérique convergente 20. La diode électroluminescente n'est pas une source cohérente, mais, par rapport aux lampes à filament, le faisceau lumineux émis est très directif et de spectre limité. La lentille sphérique 20 fait converger le faisceau source en un point A qui constitue ainsi une source sensiblement ponctuelle d'où est issu le faisceau incident FI. Comme le point A est situé à une distance supérieure à la distance focale de la lentille sphérique 21, le faisceau incident FI est légèrement convergent à la sortie du premier dispositif optique 2. Une section droite de ce faisceau incident, située entre la lentille 21 et le dioptre d'entrée 30 à 1 cm du dioptre 30, est un disque sensiblement parallèle au dioptre plan d'un diamètre de 3 cm environ. Par suite de la convergence, la section droite du faisceau réfléchi sortant par le dioptre de sortie 32, prise sur la lentille cylindrique 40, est un disque d'un diamètre sensiblement égal à 2 cm.

Outre le fait que la source lumineuse a l'avantage d'être économique et de ne pas exiger les précautions d'emploi explicitées ci-avant pour les diodes laser, elle présente un autre intérêt par rapport aux diodes laser. En effet le faisceau source de ces dernières est divergent et asymétrique. Il est de plus de section elliptique.Selon les modèles utilisés cette divergence peut varier entre 10 x 25 degrés et 30 x 40 degrés. Il faut donc utiliser des lentilles de grande ouverture pour créer une source sensiblement ponctuelle ce qui peut être pénalisant notamment pour l'encombrement.

Le Demandeur a remarqué de façon surprenante que ce caractère légèrement convergent du faisceau incident permet d'obtenir une meilleure qualité de l'image ED2. Les premiers essais du dispositif selon l'invention n'ont pas permis d'obtenir actuellement un faisceau constamment parallèle depuis la sortie de la lentille 21 jusqu'au plan de l'image ED2. Une première explication de ce phénomène serait liée au caractère faiblement cohérent de la source lumineuse S. Cependant l'obtention d'un tel faisceau parallèle n'est pas à exclure en modifiant l'agencement du premier dispositif optique 2 (notamment en plaçant le foyer objet de la lentille 21 au point A) dans le cas où la source lumineuse utilisée le permet.

On se réfère maintenant plus particulièrement à la figure 3 pour décrire le phénomène de réflexion à l'intérieur du prisme 3. L'empreinte digitale ED présente une succession de creux CED et de saillies SED. Les saillies SED sont en contact avec la surface d'appui 31 alors que les creux ne le sont pas. Au niveau des creux de l'empreinte digitale ED on obtient donc un interface verre-air alors qu'au niveau des saillies de cette empreinte digitale on obtient un interface verre-peau. L'inclinaison de la face d'appui 31, l'angle d'incidence du faisceau incident FI et l'indice de réfraction du prisme sont tels qu'une partie FI1 du faisceau incident FI arrivant sur la surface d'appui au niveau d'un interface verre-air est réfléchie totalement en un faisceau réflé chi FR1. Par contre une partie FI2 du faisceau incident FI arrivant sur la face d'appui au contact d'un interface verre-peau est en partie, voire totalement, absorbée dans la peau, créant ainsi un faisceau réfléchi FR2 ayant beaucoup moins d'intensité lumineuse que le faisceau FR1. L'ensemble de ces faisceaux FR1 et FR2 comprend un motif EDO correspondant à l'empreinte digitale ED et obtenu par réflexion du faisceau incident sur la surface d'appui. Ce motif EDO présente une anamorphose due à l'inclinaison de la surface d'appui. L'ensemble des faisceaux FR1 et FR2 après passage dans le dispositif optique 4, non représenté sur la figure 3, va former l'image ED2 qui sera une succession de zones sombres, correspondant aux saillies de l'empreinte digitale, et claires, correspondant aux creux de l'empreinte digitale.

Ainsi qu'il a été expliqué ci-avant le motif EDO correspondant à l'empreinte digitale ED présente ure anamorphose c'est-à-dire une compression dans le plan optique. La lentille cylindrique 40 a donc pour but de corriger cette anamorphose et de produire une image ED1 dans laquelle les justes proportions sont rétablies. La lentille sphérique 41 permet en combinaison avec la lentille cylindrique 40 d'effectuer d'une part la mise au point de l'image ED2 et de façon que celle-ci soit réelle et compatible avec les dimensions de la matrice CCD 50 et d'autre part de redresser le plan de l'image ED2 dans une direction tendant à le rendre pratiquement parallèle à la section droite du faisceau réfléchi qui l'atteint. Ainsi on obtient un angle $\underline{a}$ égal à environ 3,5 degrés.

La fonction du filtre spatial 42 introduit entre la lentille cylindrique et la lentille sphérique permet de limiter les aberrations de sphéricité du faisceau et permet un gain en netteté et en contraste de l'image ED2.

Ainsi, selon l'invention, la lentille cylindrique 40 et la lentille sphérique 41 constituent des moyens de correction propres à compenser l'anamorphose du motif EDO occasion née par l'inclinaison de la surface d'appui 31 et propres à redresser le plan image comme expliqué ci-avant. Compte tenu de la géométrie utilisée, on a choisi des distances focales relativement courtes pour les lentilles cylindriques 40 et sphériques 41. Ce redressement du plan de l'image à la mise au point permet donc de réduire l'inclinaison $\underline{a}$ du récepteur 5 et d'augmenter la qualité de l'image, remarque étant faite que l'on améliore encore cette qualité avec une empreinte digitale légèrement grasse.

La lentille sphérique 20 et la lentille sphérique 21 du premier dispositif optique 2 constituent des moyens de convergence propres d'une part à faire converger le faisceau incident au point A de façon à créer une source sensiblement ponctuelle et d'autre part propres à créer un faisceau incident avantageusement légèrement convergent.

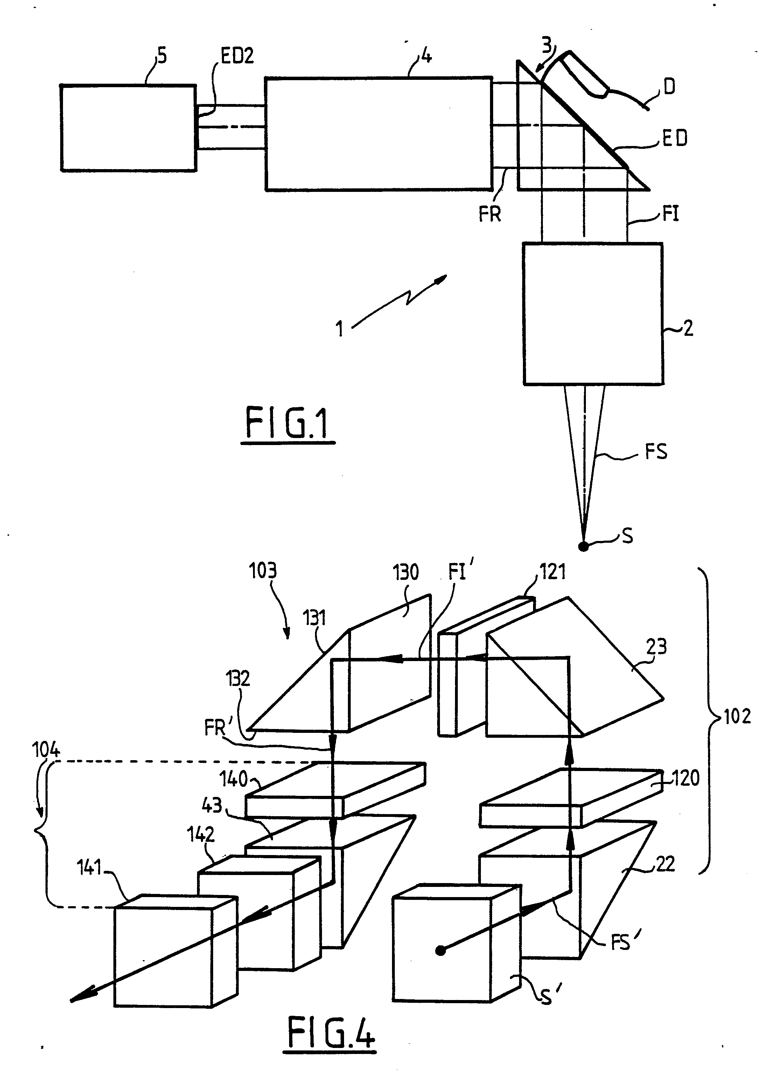

Si l'on se réfère maintenant à la figure 4, on voit que l'on a représenté un second mode de réalisation du dispositif selon l'invention permettant de loger celui-ci dans un boîtier compact. Sur cette figure les éléments analogues ou ayant des fonctions analogues à celles représentées sur la figure 2 possèdent des références, soit augmentées de 100, soit affectées d'un', par rapport à celles qu'ils avaient sur la figure 2. Seules les différences entre ces deux figures seront décrites.

Le premier dispositif optique comprend en outre un prisme à réflexion totale 22 interposé entre la source lumineuse S' et la lentille 120 ainsi qu'un autre prisme à réflexion totale 23 interposé entre les lentilles 120 et 121. Le deuxième dispositif optique 4 comprend de la même façon en outre un prisme à réflexion totale 43 interposé entre la lentille cylindrique 140 et le filtre spatial 142. Les prismes 22 et 23 constituent des premiers moyens de renvoi et le prisme 43 constitue des deuxièmes moyens de renvoi. Ces différents moyens de renvoi permettent de diriger dans l'espace les faisceaux sources incident et réfléchi selon des directions différentes afin de réduire l'encombrement des différents éléments constitutifs du dispositif. Ainsi le dispositif de la figure 4 peut-il être logé dans un boîtier cubique de 12 cm de côté non représenté sur cette figure.

L'invention peut comporter des variantes notamment les suivantes :

- le Demandeur a observé que l'utilisation d'une telle source laser crée un contraste plus important que celui occasionné par la diode électroluminescente. Dans cette application particulière d'authentification d'empreintes, le Demandeur préfère donc utiliser la diode électroluminescente qui donne un contraste moins élevé tout en étant largement suffisant car le bruit de fond de la source lumineuse est limité, et qui ne nécessite pas les précautions d'emploi ci-avant décrites. Cependant, la source lumineuse peut être constituée d'une source laser si les contraintes d'utilisation ne sont pas jugées pénalisantes,

- des lentilles de distances focales différentes peuvent être choisies, leur disposition mutuelle devant alors être modifiée;

- le récepteur n'est pas limité à une matrice CCD.

Bien entendu certains des moyens décrits ci-dessus peuvent être omis dans les variantes où ils ne servent pas.

## Revendications

1. Dispositif de formation d'une image d'une surface en relief, notamment une empreinte digitale, comprenant :

- une source lumineuse (SS') générant un faisceau source lumineux (FS,FS'),

- un premier montage optique (2,102) produisant un faisceau incident (FI,FI') à partir dudit faisceau source (FS,FS'),

- un milieu homogène transparent (3,103) possédant un dioptre d'entrée (30,130) par lequel le faisceau incident (FI,FI') pénètre à l'intérieur desdits milieux selon une direction principale d'incidence (01), une surface d'appui (31,131) sensiblement plane sur laquelle la surface en relief (ED) est en contact, cette surface d'appui étant inclinée relativement à la direction principale d'incidence de façon à réfléchir totalement une partie (FR1) du faisceau incident (FI), le faisceau réfléchi (FR,FR') sortant du milieu (3,103) selon une direction principale de réflexion (02) par l'intermédiaire d'un dioptre de sortie (32,132) et comprenant un motif (EDO) correspondant à ladite surface en relief (ED), avec une anamorphose occasionnée par l'inclinaison de la surface d'appui (31,131),

- un deuxième montage optique (4,104) recevant le faisceau réfléchi (FR,FR') et propre à former ladite image (ED2), caractérisé en ce que la source lumineuse (S,S') a un spectre étroit,

- en ce que le premier dispositif optique comporte des moyens de convergence (20,21; 120,121) propres à faire converger le faisceau source (FS,FS')

en un point constituant une source auxiliaire (A) sensiblement ponctuelle et

- en ce que le deuxième dispositif optique (4,104) comporte des moyens de correction (40,41; 140,141) propres à redres ser le plan de l'image (ED2) dans une direction tendant à le rendre parallèle à la section droite du faisceau réfléchi (FR,FR′) qui l'atteint et propres à compenser l'anamorphose dudit motif (EDO).

2. Dipositif selon la revendication 1, caractérisé en ce que le plan contenant les directions principales d'incidence (01) et de réflexion (02), dit plan optique, est sensiblement perpendiculaire à la surface d'appui (31,131) et en ce que les moyens de correction comprennent une lentille cylindrique convergente (40,140) dont l'axe géométrique longitudinal est sensiblement orthogonal audit plan optique.

3. Dispositif selon la revendication 2, caractérisé en ce que la distance focale de la lentille cylindrique (40,140) est supérieure à la distance maximale séparant son axe optique de la surface en relief (ED) et en ce que les moyens de correction comprennent en outre une lentille sphérique convergente (41,141) placée en aval de la lentille cylindrique (40,140).

4. Dispositif selon la revendication 3, caractérisé en ce que le deuxième dispositif optique comprend en outre un filtre spatial (42,142) disposé au niveau du segment de convergence de la lentille cylindrique (40,140).

5. Dispositif selon l'une des revendications 1 à 4, caractérisé en ce que les moyens de convergence comprennent une première lentille sphérique convergente (20,120) propre à faire converger le faisceau source (FS,FS′) au point constituant la source auxiliaire (A), cette première lentille sphérique convergente étant suivie d'une deuxième lentille sphérique convergente (21,121) située à une distance de la source auxiliaire (A) au moins égale à sa distance focale.

6. Dispositif selon l'une des revendications précédentes, caractérisé en ce que le milieu homogène transparent est un prisme de section droite triangulaire rectangle (3,103), dont les deux côtés perpendiculaires sécants constituent les dioptres d'entrée (30,130) et de sortie (32,132) et dont le côté joignant les deux côtés perpendiculaires sécants constitue la surface d'appui (31,131).

7. Dispositif selon l'une des revendications précédentes, caractérisé en ce que la source lumineuse est une diode électroluminescente.

8. Dispositif selon l'une des revendications précédentes, caractérisé en ce que le premier dispositif optique (102) comprend des premiers moyens de renvoi du faisceau lumineux le traversant.

9. Dispositif selon l'une des revendications précédentes, caractérisé en ce que le deuxième dispositif optique (104) comprend des deuxièmes moyens de renvoi du faisceau réfléchi le traversant.

10. Dispositif selon les revendications 6 à 9, caractérisé en ce qu'il est logé dans un boîtier compact.

FIG.2

FIG.3

## FIG.1

## FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | DE-A-3 421 220 (G. MULL)<br>* figure 3 *<br>--- | 1,6 | G 06 K 9/00<br>A 61 B 5/103 |
| A | EP-A-0 045 913 (SIEMENS AG)<br>* figure 1 *<br>--- | 1 | |
| A | EP-A-0 045 915 (SIEMENS AG)<br>* figure 1 *<br>----- | 1,3,7 | |

DOMAINES TECHNIQUES
RECHERCHES (Int. Cl.5)

A 61 B 5/103
G 06 K 9/00

Le présent rapport a été établi pour toutes les revendications

| Lieu de la recherche | Date d'achèvement de la recherche | Examinateur |
|---|---|---|
| BERLIN | 10-01-1990 | ZOPF K |

EPO FORM 1503 03.82 (P0402)